# EUROPEAN PATENT SPECIFICATION

(11) **EP 2 083 899 B1**
(45) Date of publication and mention of the grant of the patent: **02.11.2011**
(21) Application number: 07867428.0
(22) Date of filing: 15.11.2007
(51) Int. Cl.: A61M 25/09, A61M 25/01, A61M 25/00, A61B 17/22

(54) **MULTI-DIMENSIONAL LOOP TIP ELONGATE WIRE GUIDE**
LÄNGLICHER MEDIZINISCHE MEHRDIMENSIONALE SCHLAUFENSPITZENSFÜHRUNGSDRAHT
FIL GUIDE ALLONGÉ DONT L'EXTRÉMITÉ COMPORTE UNE BOUCLE MULTIDIMENSIONNELLE

(30) Priority: 15.11.2006 US 859208 P
(43) Date of publication of application: 05.08.2009
(73) Proprietor: Cook Medical Technologies LLC, Bloomington, IN 47402 (US)
(72) Inventor: HARDIN, David M., Winston-Salem, NC 27106 (US); MCLAREN, Douglas E., Winston-Salem, NC 27106 (US)
(74) Representative: Garratt, Peter Douglas
(86) International application number: PCT/US2007/023810
(87) International publication number: WO 2008/060529

(56) References cited:
- WO-A-2006/039216
- WO-A-2006/066114
- US-A1- 2002 183 783
- US-A1- 2005 027 245
- US-A1- 2006 074 442

## Description

### FIELD OF THE INVENTION

The present invention relates to multi-dimensional loop tip elongate medical devices.

### BACKGROUND OF THE INVENTION

Wire guides are elongate flexible members used to provide a path along which another medical device can be moved. The path provided by the wire guide can be used to navigate another medical device, such as a catheter, through a body vessel. The use of wire guides to define such a path is known in the art. Briefly, a wire guide is navigated through a body vessel toward a point of treatment. Once positioned within the vessel, a second medical device, frequently a cannula such as a catheter, is placed over the wire guide and moved along its length toward the point of treatment. Thus, the wire guide provides an established path for placing other devices, thereby eliminating the burden of performing navigation procedures for each device passed into the vessel.

During placement of a wire guide, an operator must navigate the wire guide through various body lumens. Often, the lumen defines a torturous path due to the presence of natural bends and/or curves, natural impediments such as tiny fronds that line the bile and pancreatic ducts, or unnatural impediments, such as tumors, build-ups, and/or strictures. The presence of a torturous path may make navigation of a wire guide difficult. For example, the presence of an impediment may block the wire guide from navigating further into the vessel.

The prior art contains many examples of wire guides having straight flexible tips intended to aid in the navigation around such impediments. The presence of a straight flexible tip, however, may in fact make navigation more difficult. For example, upon encountering an impediment, the straight flexible tip may bend toward one of the vessel walls, which may result in unintended contact between the tip and vessel wall. This situation may lead to undesirable effects in the vessel wall. Further, the straight tip may bend and tum back upon itself upon encountering the impediment. This formation of an unstable turn in the wire guide makes further navigation difficult.

Additionally, the prior art also contains single loop tip wire guides which may offer improved flexibility over wire guides with straight flexible tips. One example of a single loop tip wire guide is disclosed in pending U.S. Publication No. 2004-0215208, entitled "Loop Tip Wire-guide," filed November 21, 2003, which claims priority to U.S. Provisional Application Serial No. 60/430,466, filed December 2, 2002. In this device, a resilient loop positions, and a closure member affixes, the distal end of a wire-guide relative to the wire-guide. When this device is navigated through a body vessel and encounters an impediment, the distal end of the wire- guide does not move relative to the remainder of the wire-guide due to the presence of the loop and closure member. Instead, the loop deforms in response to the impediment. The resiliency of the loop creates a force opposing the impediment and directs the loop away from the impediment. This defines a path for the remainder of the wire-guide to follow and enables the wire-guide to navigate about the impediment and continue along the interior of the vessel. Reference is also directed to WO 2006/039216 which discloses a steerable wire guide with a single loop.

Document US 2006/0074442 A1 describes a device having an elongate hollow guidewire body (14) with proximal end, a deflectable distal end (39), and an axial lumen between the ends. A rotating drive shaft is positioned at or near the distal end of the guide wire body and extending through the axial lumen of the hollow guidewire body. The drive shaft is actuated to create a passage through an occlusive or stenotic material in body lumen.

Document WO 2006/039216 A2 describes a steerable wire-guide (10) comprises a longitudinal axis and an elongate member (12). The elongate member comprises a leading portion and a body portion. The leading portion comprises a loop (22) and the body portion comprises a firstwire (26) and a second wire (28). The first and second wire are movable relative to each other such that relative movement of the first wire with respect to the second wire directs the leading portion in a first direction, which is at an angle relative to the longitudinal axis, and relative movement of the second wire with respect to the first wire directs the leading portion in a second direction different from the first direction.

Notwithstanding the improved flexibility attained from a single loop tip wire guide, additional embodiments of a loop tip wire-guide may be desirable in certain applications. For example, if the point of treatment is located in a side branch or beyond a bifurcation of the main vessel, it would be desirable if a loop tip wire-guide provided the user with the greater ability to direct the loop tip of the wire-guide through a main body vessel and into the side branch vessel or bifurcation.

In view of the drawbacks of current technology, there is an unmet need for an improved device that can effectively maneuver through tortuous body lumens.

### BRIEF SUMMARY OF THE INVENTION

Accordingly, a multi-dimensional loop tip structure is provided.

The present invention is set forth in the appended claims.

### BRIEF DESCRIPTION OF THE DRAWINGS

Figure 1 is a side view of a double loop tip structure according to the embodiment;

Figure 2 is an end view of Figure 1 from the distal end of the loop tip structure;

Figure 3 is a side view of a double loop tip structure;

Figure 4 is a side view of the double loop tip structure of Figure 3 in which the proximal end of the second loop is attached to the core member;

Figure 5 is a side view of the double loop tip structure in which the core wires are unbraided and inserted through a closure member;

Figure 6 is a cross-séctional view of a double loop tip structure in which legs are attached to a core member;

Figure 7 is a cross-sectional view of a medical elongate structure in which the legs are not attached to each other;

Figure 8 is a side view of a medical elongate structure comprising a plurality of loops;

Figure 9 is an end view of the medical elongate structure of Figure 8;

Figure 10 is a side view of the double loop tip structure of Figure 3 in which the proximal end of the second loop is attached to the proximal end of the first loop;

Figure 11 is a side view of a loop tip structure formed by crossing a middle section of a wire on itself and braiding the ends of the loop along a longitudinal axis of the loop tip structure; and

Figure 12 is a side view of a double loop tip structure in which the first and second core members are inserted within a closure member.

### DETAILED DESCRIPTION OF THE INVENTION

The embodiments are described with reference to the drawings in which like elements are referred to by like numerals. The relationship and functioning of the various elements of the embodiments are better understood by the following detailed description. However, the embodiments as described below are by way of example only, and the invention is not limited to the embodiments illustrated in the drawings. It should also be understood that the drawings are not to scale and in certain instances details have been omitted, which are not necessary for an understanding of the embodiments, such as conventional details of fabrication and assembly.

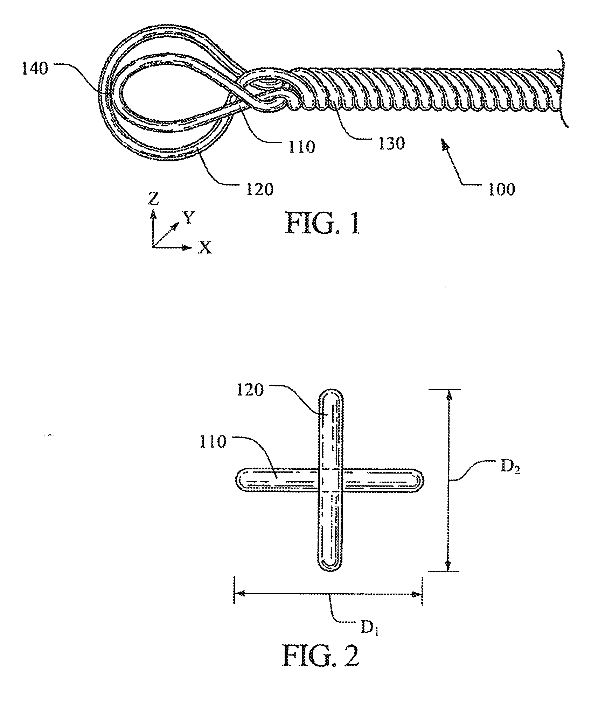

Figure 1 illustrates the distal portion of a double loop tip wire guide 100 according to a first embodiment. The term "loop" as used herein refers to a completely bounded or partially bounded structure. The double loop tip wire guide 100 comprises a first loop 110 and a second loop 120. The first loop 110 and second loop 120 are configured to navigate through tortuous body lumens and stricture-containing body lumens. Generally, the first loop 110 and second loop 120 are configured to navigate pass such impediments by deflecting off the impediment to define a path for the remainder of the wire guide 100 to follow.

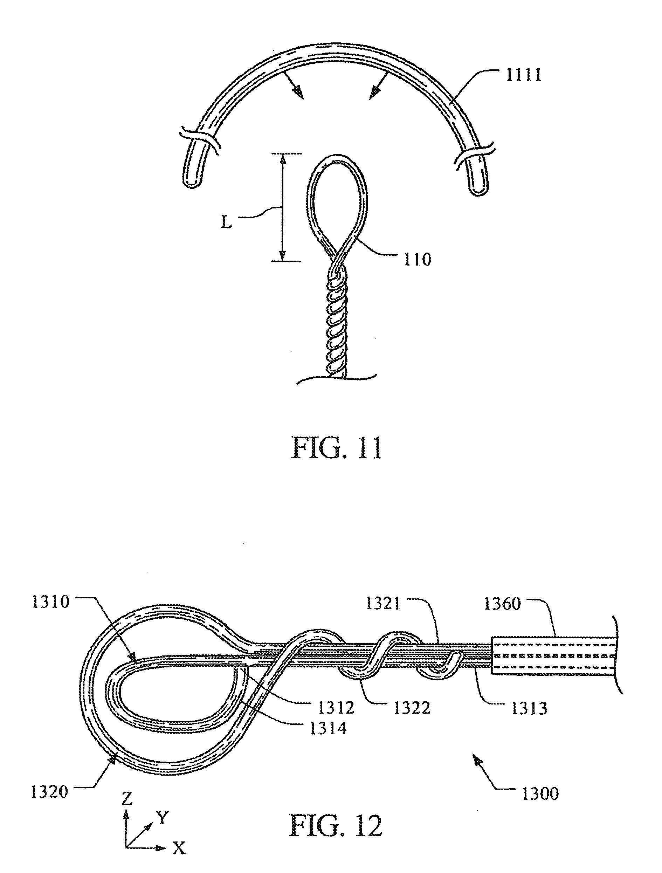

Figure 11 shows that the first loop 110 may be formed by bending a middle portion of a core wire 111 until the core wire 111 crosses over on itself. The location at which the core wire 111 crosses over on itself determines the size of the first loop 110, L, as shown in Figure 11. After the first loop 110 is formed, the balance of the core wire 111 may be twisted or braided for the purpose of securing the first loop 110. Figure 1 shows that the first loop 110 is formed within the x-y plane, where the x-axis is defined as parallel to the longitudinal axis of the wire guide 100.

After the first loop 110 is formed, the second loop 120 may be formed as follows. A second core wire may be bent over the first loop 110 in the x-z plane. Similar to the formation of the first loop 110 as shown in Figure 11, the second core wire is bent until the wire crosses over on itself. After formation of the second loop 120, the end portions of the second core wire are twisted to ensure that the second loop 120 remains in its loop-formed shape. Figure 1 shows that the second loop 120 is formed within the x-z plane, where the z-axis is defined as perpendicular to the longitudinal axis of the wire guide 100. As shown in Figure 1, each of the core wires of the first and second loops 110, 120 have been twisted together to form a resultant braided core wire 130. In addition, Figure 1 shows that the loops 110 and 120 are positioned substantially perpendicular relative to each other.

The interbraiding of each of the core wires of the loops 110, 120 may provide the primary or only means of attachment for the loops 110, 120. This provides a flexible double loop tip wire guide 100 structure that is capable of navigating pass impediments contained in a body lumen. The resiliency of the loops 110, 120 creates a force opposing the impediment in the body lumen and forces the loops 110, 120 away from the impediment, thereby defining a path for the braided core wire 130 to follow. Because the double loop tip wire guide 100 contains loops 110, 120 in both the x-y and x-z planes, the wire guide 100 is capable of constantly being self-aligned to the center of a body lumen when encountering impediments therein. This is advantageous over a single loop tip wire guide, which has limited balancing and centering capabilities. The first and second loops 110, 120 need not be connected at location 140, as shown in Figure 1.

Although the first and second loop 110, 120 are shown configured substantially perpendicular relative to each other, they may be configured at various other angular configurations relative to each other. The loops 110, 120 may also have a variety of shapes, including circular and tear-dropped.

As an alternative to Figure 1, a single core wire may be used to form both the fist and second loops 110, 120 by splitting the distal portion of the core wire into two wires. Each of the wires would be bent back upon itself to form a separate loop.

Figure 2 is an end view of Figure 1 at the distal end of the double loop tip wire guide 100. Figure 2 illustrates that the first loop 110 resides in the x-y plane and the second loop 120 resides in the x-z plane. D₁ represents the diameter of the first loop 110 as measured from the outer edge of the first core wire. Similarly, D₂ represents the diameter of the second loop 120 as measured from the outer edge of the second core wire. The term 'diameter' refers to the distance as measured along a direction perpendicular to the longitudinal axis of the wire guide 100. D₁ and D₂ may range between about 3 mm and about 6 mm, and more preferably between about 4 mm and about 5 mm.

Figure 12 is an alternative double loop tip wire guide 1300. The double loop tip wire guide 1300 comprises a first loop 1310 and a second loop 1320, the second loop 1320 being perpendicular to the first loop 1310. The first loop 1310 resides in the x-y plane and the second loop 1320 resides in the x-z plane. The first loop 1310 is formed by bending a predetermined distal portion 1311 of the first core wire 1313 onto itself. After bending the distal portion 1311 to form the first loop 1310, the distal portion 1311 is attached to the first core wire 1313 at location 1312 to maintain the loop configuration. The location 1312 at which the distal portion is attached to the first core wire 1313 determines the size of the first loop 1310. Numerous factors govern the size of the first loop 1310, including the body lumen the wire guide 1300 is to be navigated through and the tortuosity of the body lumen. A variety of means known to one of ordinary skill in the art may be used to attach the distal portion 1311 to the first core wire 1313, including soldering, welding, or a mechanical attachment. Unlike the double loop tip wire guide 100 shown in Figure 1, the first core wire 1313 may remain unbraided, as shown in Figure 12.

The second loop 1320 is formed as follows. The distal end of the second core wire 1321 is bent back on itself over the first loop 1310 to form the second loop 1320. The second loop 1320 is shown to reside in the x-z plane and is substantially perpendicular to the first loop 1310. After formation of the second loop 1320, the distal end 1322 of the second loop 1320 is twisted over the first and second core wires 1313, 1321 to secure the second loop 1320 in its loop-formed shape.

Any biocompatible material may be used to form the double loop tip wire guide 100 shown in Figure 1. These materials include polymers, stainless steel, shape memory alloys, and any combinations thereof. Alternatively, the wire guide 100 may comprise a composite structure in which the core wire is formed from a biocompatible polymeric material and the loops are formed from a shape memory alloy.

The double loop tip structure may preferably be formed by bending the wire over a mandrel. Preferably, the double loop tip feature is formed from a shape memory alloy such as nitinol. Nitinol is a nickel-titanium alloy that may undergo a substantially reversible phase transformation that allows it to "remember" and return to a previous shape or configuration. For example, a transformation between an austenitic phase and a martensitic phase may occur by cooling and/or heating (shape memory effect) or by isothermally applying and/or removing stress (superelastic effect). Austenite is characteristically the stronger phase and martensite is the more easily deformable phase.

In an example of the shape memory effect, nitinol having an initial configuration in the austenitic phase may be cooled below a transformation temperature (M_{f}) to the martensitic phase and then deformed to a second configuration. Upon heating to another transformation temperature (A_{f}), the material may spontaneously return to its initial configuration. In another example in which the superelastic effect is utilized, stress may be applied to a shape memory material having an initial shape in the austenitic phase to cause a transformation to the martensitic phase without a change in temperature. A return transformation to the austenitic phase may be achieved by removing the applied stress. The superelastic effect may be exploited at a temperature above A_{f}.

Still referring to Figure 12, a closure member 1360 may be provided. The closure member 1360 is a hollow tubular member through which the untwisted portions of the first and second core wires 1313, 1321 are inserted. The closure member 1360 may assist in preventing the first and second core wires 1313, 1321 from separating from each other during navigation of the wire guide 1300 within a body lumen. Alternatively, the closure member 1360 may comprise lumens through which the distal ends of the first and second core wires 1313, 1321 are inserted.

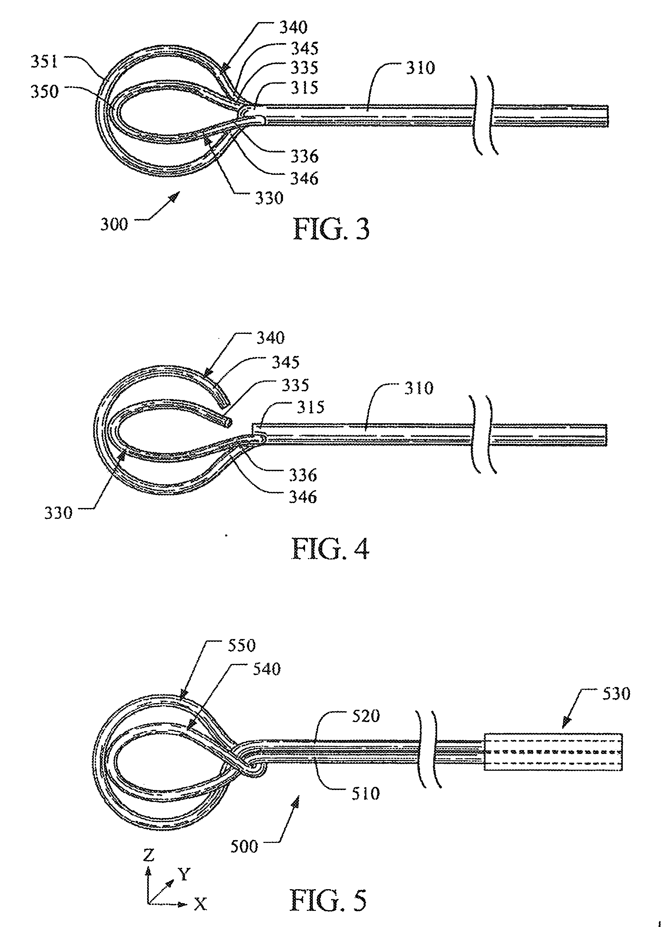

Figure 3 is an alternative examplary double loop tip wire guide 300. The double loop tip wire guide 300 comprises a first loop 330 and a second loop 340. The first and second loops 330, 340 are preformed, and then the proximal end 336 of the first loop 330 is affixed to the distal end 315 of core wire 310. The proximal end 346 of the second loop 340 is affixed to the proximal end 336 of the first loop 330. Other variations of attaching the preformed loops 330, 340 to the distal end 315 of the core wire 310 are contemplated. For example, the proximal end 346 of the second loop 340 can be connected to the distal end 315 of the core wire 310, as shown in Figure 4. The first and second loops 330, 340 may be affixed by a variety of ways as known to one of ordinary skill in the art, including welding or through a mechanical attachment. The loops 330, 340 may be preformed in a number of ways as would be apparent to one of ordinary skill in the art. For example, each of the loops 330, 340 may be molded into the desired loop-shaped structure. Alternatively, each of the loops 330, 340 may be heat set into the desired loop-shaped configuration.

As Figure 3 illustrates, only the proximal ends 336, 346 of the first and second loops 330, 340 are attached to each other and the distal end 315 of the core wire 310. The distal end 335 of the first loop 330 need not completely fold back on itself such the distal end 335 is contacting the proximal end 336 of the first loop 330. Similarly, the distal end 345 of the second loop 340 need not completely fold back on itself such that the distal end 345 is contacting the proximal end 346 of the second loop 340. Additionally, the distal ends 335, 345 of the first and second loops 330, 340 are shown not to be connected to each other, and the apex 350 of the first loop 330 is shown not to be connected to the apex 351 of the second loop 340. Minimizing the points of attachment between the first and second loops 330, 340 may enhance the flexibility and resiliency of the wire guide 300.

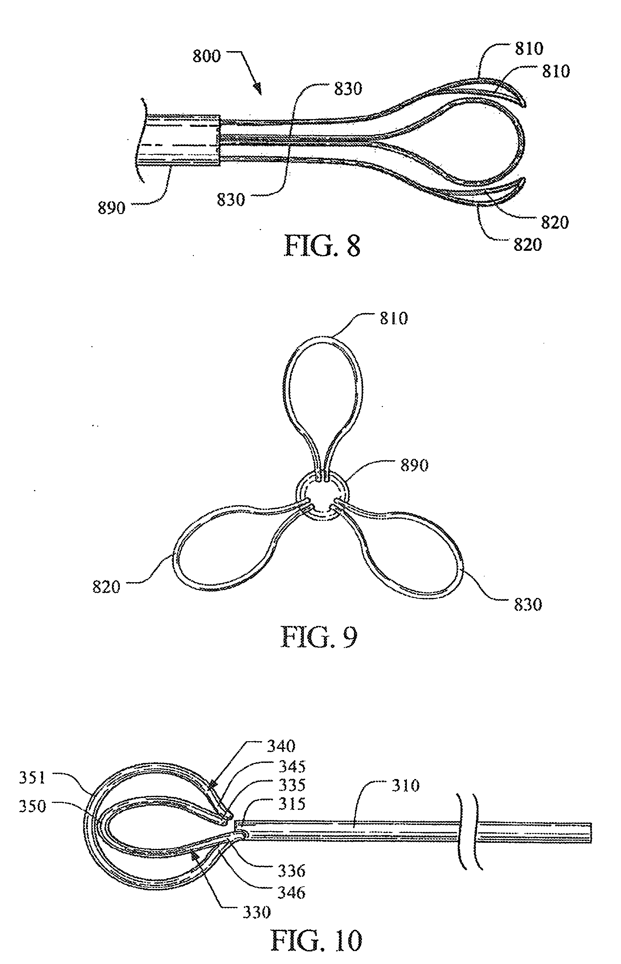

Alternatively, it may be advantageous in certain applications to utilize a double loop tip wire guide that is more rigid by, for example, attaching the apexes 350 and 351 to each other and completely folding back the distal ends 335, 345 to form fully closed first and second loops 330, 340. As another example, Figure 10 illustrates a double loop-tip structure in which the distal end 345 of the second loop 340 and the distal end 335 of the first loop 330 are each folded back on themselves so as to form complete loops. Additionally, the distal ends 335, 345 are connected to each other and affixed to the distal end 315 of core wire 310. The apexes 350 and 351 are also shown to be connected. Maximizing the points of attachment between the first and second loops 330, 340 helps to create a relatively more rigid loop-shaped structure as compared to the wire guide 300 of Figure 3. There are numerous factors that determine whether a relatively more rigid or less rigid loop-shaped structure is required, including the tortuosity of the body lumen that the wire guide is to be navigated through and the types of impediments within the body lumen. The double loop tip feature may be formed unitarily with the remainder of the device at the time of manufacture.

The double loop tip feature described with respect to Figures 3, 4, and 10 applies to various other medical devices. For example, a double loop tip catheter in accordance with the examples of Figures 3, 4, and 10 is contemplated. A double loop tip catheter would allow the tip of such a catheter to maneuver through tortuous body lumens having impediments therein without collapsing on itself.

Figure 5 shows another example of a double loop tip wire guide 500. The double loop tip wire guide 500 comprises a first loop 540 and a second loop 550. Similar to the wire guide 100 of Figure 1, the first loop 540 may be formed by bending a middle portion of a first core wire 510 until the core wire 510 crosses over on itself. The location at which the core wire 510 crosses over on itself determines the size of the first loop 540 (See Figure 11). The first loop 540 is shown to be formed within the x-y plane, where the x-axis is defined as parallel to the longitudinal axis of the wire guide 500. After the first loop 540 is formed, the balance of the first core wire 510 is shown as remaining unbraided. The untwisted portion of the distal end of the first core wire 510 is inserted into a closure member 530 to maintain the shape of the first loop 540.

After the first loop 540 is formed, the second loop 550 may be formed as follows. A second core wire 520 is bent over the first loop 540 in the x-z plane. Similar to the formation of the first loop 540, the second core wire 520 is bent until the wire 520 crosses over on itself. After the second loop 550 is formed, the balance of the second core wire 520 is shown as remaining unbraided. The untwisted portion of the distal end of the second core wire 520 is inserted into the closure member 530 to maintain the shape of the second loop 540.

As shown in Figure 5, the closure member 530 provides the sole means of securing the distal ends of the first and second core wires 510 and 520. Various parts of the first and second loops 540, 550 may also be attached to each other to create a more rigid structure if desired. Insertion of the untwisted portions of the distal ends of the first and second core wires 510, 520 within the closure member 530 prevents the wires 510, 520 from separating from each other during navigation of the wire guide 500 within a body lumen. The closure member 530 is shown to extend along the proximal end of the wire guide 500 so that only a portion of the first and second core wires 510, 520 reside within the closure member 530. The closure member 530 may extend along any portion of the first and second core wires 510, 520. Alternatively, the closure member 530 may extend the entire length of the core wires 510, 520 such that the entire untwisted portions of the first and second core wires 510, 520 are within the closure member 530.

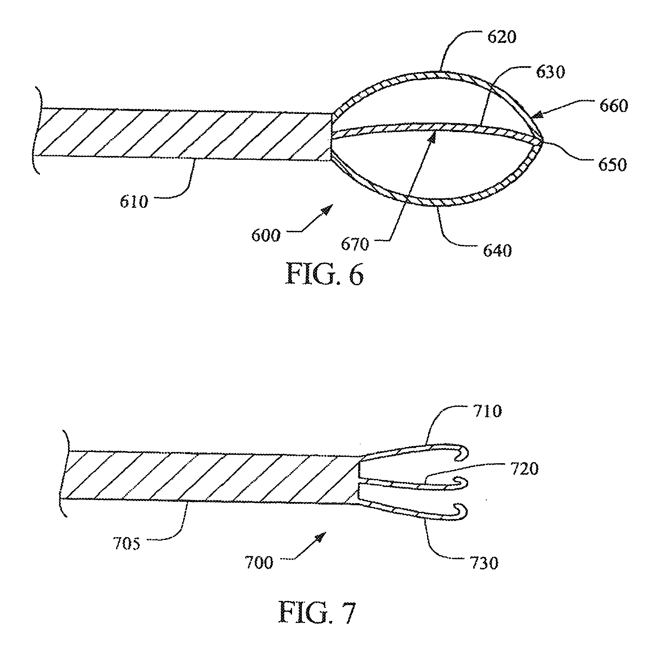

There have been described examplary double loop tip structures in which the loops are planar and reside in an x-y or x-z plane. Multi-loop tip wire guides in which the loops are nonplanar are also described. Figure 6 is an example of a multi-loop tip wire guide 600 comprising a first nonplanar loop 660 and a second nonplanar partial loop 670. The first loop 660 and second partial loop 670 are oriented adjacent to each other. The distal end of the core wire 610 splits into three wires or legs 620, 630, and 640. The legs 620, 630, and 640 are defined as distinct elongate structures which extend away from a distal end of the core wire 610. The legs 620, 630, and 640 may be spaced away from the longitudinal axis of the core member. Two legs may be connected at their distal ends to form a loop. Figure 6 shows that legs 620 and 640 are connected to each other at their distal ends (apex 650) so as to form the first loop 660. Leg 630 has an arch-like structure that bows outward. Leg 630 connects to the distal ends of wires 620 and 640 at apex 650 so as to form the second partial loop 670. Although not shown, the distal end of the core wire 610 may also split into four or more legs that define three or more nonplanar or partial loops.

In certain applications, it may be desirable for the distal end of the legs to not be attached to each other. Figure 7 shows such an examplary wire guide 700 in which legs 710, 720, 730 are not connected to each other. The distal end of the core wire 705 splits into the three legs 710, 720, 730. Each of the distal ends of the legs 710, 720, 730 curl inwards to produce atraumatic ends. The legs 710, 720, 730 may be placed at various positions with respect to each other. For example, the legs 710, 720, 730 may be circumferentially disposed symmetrically around the distal end of the core wire 705 at 120 degree intervals. Although three legs are shown, the distal end of the core wire 705 may split into four or more legs. The legs that have been described in Figures 6 and 7 need not split from a core wire. Rather, the legs may be subassembled components that are affixed to the core wire by any method known to one of ordinary skill in the art, including welding or through a mechanical attachment.

Figures 8-9 show another example of a wire guide 800. A core wire 890 splits into three wires 810, 820, and 830. Each of the three wires 810, 820, 830 is bent back on itself such that both ends of each of the wires are connected to the core wire 890. As Figures 8-9 show, the ends of each of the wires 810, 820, 830 do not cross to form closed loops. The wires 810, 820, 830 are not connected at the apex 891 to enable the wire guide 800 to be flexible. The resultant wire guide 800 resembles a whisk-like structure having multiple loops or lobes. The loops or lobes are shown as nonplanar. Although Figure 8 shows a core wire 890 splitting into the wires 810, 820, 830, the whisk-like structure may be subassembled separately and subsequently attached to the distal end of the core wire 890 by any means known to one of ordinary skill in the art, including welding or soldering.

The above-described double loop tip structures may be treated with a hydrophilic coating or hybrid polymer mixture, such as those based on polyvinyl puroladine and cellulose esters in organic solvent solutions. These solutions make the wire guide particularly lubricious when in contact with body fluids, which aids in navigation. As an example, the wire guides may be coated with a low friction material such as silicone, or with a hydrophilic material which becomes slippery in use in a patient. The coating may be applied by dipping, molding, or spraying a suitable coating material, such as polytetrafluoroethylene, urethane, and/or other polymeric coatings, directly to the body portion 170. The hydrophilic material may comprise a hydrophilic polymer selected from the group comprising polyacrylate, copolymers comprising acrylic acid, polymethacrylate, polyacrylamide, poly(vinyl alcohol), poly(ethylene oxide), poly(ethylene imine), carboxymethylcellulose, methylcellulose, poly(acrylamide sulphonic acid), polyacrylonitrile, poly(vinyl pyrrolidone), agar, dextran, dextrin, carrageenan, xanthan, and guar. The hydrophilic polymers can also include ionizable groups such as acid groups, e.g., carboxylic, sulphonic or nitric groups. The hydrophilic polymers may be cross-linked through a suitable cross-binding compound. The cross-binder actually-used depends on the polymer system: If the polymer system is polymerized as a free radical polymerization, a preferred cross-binder comprises 2 or 3 unsaturated double bonds.

The above figures and disclosure are intended to be illustrative and not exhaustive. This description will suggest many variations and alternatives to one of ordinary skill in the art. All such variations and alternatives are intended to be encompassed within the scope of the attached claims. Those familiar with the art may recognize other equivalents to the specific embodiments described herein which equivalents are also intended to be encompassed by the attached claims.

## Claims

1. A loop tip wire guide comprising:
a first loop comprising a first core member, the first core member located proximally of the first loop, the first loop residing in a first plane parallel to a central axis of the first core member; and
a second loop comprising a second core member, the second core member located proximally of the second loop, the second loop residing in a second plane parallel to a central axis of the second core member, the second plane being oriented at a predetermined angle from the first plane;
wherein the first core member is braided to the second core member.

2. The loop tip wire guide of claim 1, wherein the predetermined angle is about ninety degrees.

3. The loop tip wire guide of claim 1, wherein the first loop comprises a first apex along a central portion thereof and the second loop comprises a second apex along a central portion thereof, wherein the first apex of the first loop is not attached to the second apex of the second loop.

4. The loop tip wire guide of claim 1, wherein the first loop comprises a first distal end, the first distal end being attached to the first core member.

5. The loop tip wire guide of claim 1, wherein the second loop comprises a second distal end, the second distal end being wrapped around the first core member and the second core member.

6. The loop tip wire guide of claim 5, wherein the first core member and the second core member comprise unbraided portions, the unbraided portions being inserted within a closure member.

## Patentansprüche

1. Schleifenspitzenführungsdraht, umfassend:
eine erste Schleife, die ein erstes Kernglied umfasst, wobei das erste Kernglied proximal der ersten Schleife positioniert ist, wobei die erste Schleife in einer parallel zu einer mittleren Achse des ersten Kernglieds verlaufenden ersten Ebene liegt; und
eine zweite Schleife, die ein zweites Kernglied umfasst, wobei das zweite Kernglied proximal der zweiten Schleife positioniert ist, wobei die zweite Schleife in einer parallel zu einer mittleren Achse des zweiten Kernglieds verlaufenden zweiten Ebene liegt, wobei die zweite Ebene in einem Vorbestimmten Winkel zur ersten Ebene ausgerichtet ist, wobei das erste Kernglied mit dem zweiten Kernglied verflochten ist.

2. Schleifenspitzenführungsdraht nach Anspruch 1, wobei der vorbestimmte Winkel ca. 90 Grad beträgt.

3. Schleifenspitzenführungsdraht nach Anspruch 1, wobei die erste Schleife einen ersten Scheitel entlang einem mittleren Teil davon umfasst und die zweite Schleife einen zweiten Scheitel entlang einem mittleren Teil davon umfasst, wobei der erste Scheitel der ersten Schleife nicht an dem zweiten Scheitel der zweiten Schleife befestigt ist.

4. Schleifenspitzenführungsdraht nach Anspruch 1, wobei die erste Schleife ein erstes distales Ende umfasst, wobei das erste distale Ende an dem ersten Kernglied befestigt ist.

5. Schleifenspitzenführungsdraht nach Anspruch 1, wobei die zweite Schleife ein zweites distales Ende umfasst, wobei das zweite distale Ende um das erste Kernglied und das zweite Kernglied herum gewickelt ist.

6. Schleifenspitzenführungsdraht nach Anspruch 5, wobei das erste Kernglied und das zweite Kernglied ungeflochtene Teile umfassen, wobei die ungeflochtenen Teile in einem Verschlussglied eingesetzt sind.

## Revendications

1. Fil-guide à pointe en forme de boucle, comprenant:
une première boucle comprenant un premier organe de coeur, le premier organe de coeur étant situé du côté proximal de la première boucle, la première boucle se situant dans un premier plan parallèle à un axe central du premier organe de coeur ; et
une deuxième boucle comprenant un deuxième organe de coeur, le deuxième organe de coeur étant situé du côté proximal de la deuxième boucle, la deuxième boucle se situant dans un deuxième plan parallèle à un axe central du deuxième organe de coeur, le deuxième plan étant orienté suivant un angle prédéterminé par rapport au premier plan ;
le premier organe de coeur étant tressé avec le deuxième organe de coeur.

2. Fil-guide à pointe en forme de boucle selon la revendication 1, dans lequel l'angle prédéterminé est d'environ quatre-vingt-dix degrés.

3. Fil-guide à pointe en forme de boucle selon la revendication 1, dans lequel la première boucle comprend un premier sommet le long d'une portion centrale de celle-ci, et la deuxième boucle comprend un deuxième sommet le long d'une portion centrale de celle-ci, le premier sommet de la première boucle n'étant pas attaché au deuxième sommet de la deuxième boucle.

4. Fil-guide à pointe en forme de boucle selon la revendication 1, dans lequel la première boucle comprend une première extrémité distale, la première extrémité distale étant attachée au premier organe de coeur.

5. Fil-guide à pointe en forme de boucle selon la revendication 1, dans lequel la deuxième boucle comprend une deuxième extrémité distale, la deuxième extrémité distale étant enveloppée autour du premier organe de coeur et du deuxième organe de cour.

6. Fil-guide à pointe en forme de boucle selon la revendication 5, dans lequel le premier organe de coeur et le deuxième organe de coeur comprennent des portions non tressées, les portions non tressées étant insérées dans un organe de fermeture.
